(19) Europäisches Patentamt
European Patent Office
Office européen des brevets

(11) EP 0 621 257 B1

(12) **FASCICULE DE BREVET EUROPEEN**

(45) Date de publication et mention de la délivrance du brevet: **30.07.1997 Bulletin 1997/31**

(51) Int. Cl.[6]: **C07C 69/24**, C07C 69/587, C07C 67/347, C07C 51/36, C07C 53/126, C07C 57/03

(21) Numéro de dépôt: **94400846.5**

(22) Date de dépôt: **18.04.1994**

(54) **Nouveaux composés di-ramifiés et leurs procédés de préparation**

Doppelt verzweigte Verbindungen und Verfahren zu deren Herstellung

Doubly branched compounds and methods of making them

(84) Etats contractants désignés:
**BE DE ES GB IT NL**

(30) Priorité: **23.04.1993 FR 9304923**

(43) Date de publication de la demande:
**26.10.1994 Bulletin 1994/43**

(73) Titulaire: **INSTITUT FRANCAIS DU PETROLE**
**92502 Rueil-Malmaison (FR)**

(72) Inventeurs:
- **Stern, Robert**
  **F-75017 Paris (FR)**
- **Kotoko, Abakar**
  **Kousseri (CM)**
- **Hillion, Gérard**
  **F-95220 Herblay (FR)**
- **Chauvin, Yves**
  **F-78230 Le Pecq (FR)**

(56) Documents cités:
**WO-A-91/11426** **WO-A-91/11428**
**FR-A- 2 202 727** **US-A- 4 318 860**

- **TETRAHEDRON LETTERS, vol.24, no.38, 1983, OXFORD GB pages 4139 - 4142 J. BLUM ET AL. 'Catalytic hydrogenation of olefins, acetylenes and arenes by rhodium trichloride and Aliquat-336 under phase transfer conditions.'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.14.11/3.4

## Description

L'invention concerne une nouvelle famille de composés chimiques issus des corps gras, caractérisés par la présence le long de la chaîne linéaire de deux ramifications. Ces composés sont obtenus par addition d'éthylène sur des composés polyinsaturés. L'invention concerne aussi le procédé d'obtention de ces composés par l'utilisation, en présence d'éthylène et de certains corps gras, de complexes anioniques du rhodium.

Enfin elle concerne l'obtention des dérivés saturés des composés diramifiés par hydrogénation partielle ou totale des composés primaires.

On sait depuis plus de 30 ans (brevet US-A-3502738) que l'on peut faire réagir l'éthylène sur différents diènes. Avec le butadiène, on obtient l'hexadiène-1,4, avec l'isoprène, le 3 méthylhexadiène et avec le pipérylène, le 2-vinylpentène, ceci avec des complexes de rhodium variés. On a aussi fait réagir l'éthylène sur des diènes dont les deux doubles liaisons peuvent-être à l'intérieur d'une chaîne, une ou deux extrémités étant substituées par des groupes alcoxy ou halogènes (Brevet US-A-3742080).

On sait aussi que l'on peut faire réagir l'éthylène ou le propylène sur des corps gras diéniques, soit de type vinyl-octadécénoate si l'on additionne un groupement éthylène, conjugués ou non, pour former des composés à une ramification, soit de type isobutyl-octadécénoate si l'on additionne deux groupements éthylène, soit de type isohexényl-octadécénoate si l'on additionne trois groupements éthylène au corps gras ; le corps gras utilisé est généralement un ester linoléique conjugué.

L'addition d'éthylène qui permet d'obtenir ces dérivés monoramifiés est décrite dans la demande de brevet internationale WO-91/11428 A1 et les composés hydrogénés monoramifiés dans la demande de brevet internationale WO-91/11426 A1.

On connaît d'autre part des dérivés éthyléniques obtenus il y a plus de 20 ans par D.G. CHASIN et coll. dans une réaction de Wittig sur une cétone située en position 10. Par hydrogénation du composé éthénylé obtenu, l'auteur fabrique le 10-éthyl stéarate d'alkyle correspondant (Chem. Phys. Lipids 6 8-30 (1971)).

On connaît en outre des composés de type diméthylstéarate, obtenus par isomérisation squelettale mais présents au côté de très nombreux autres isomères.

En revanche, il n'a pas été décrit antérieurement d'exemples de dérivés de corps gras présentant deux ramifications à 2 atomes de carbone chacune.

L'invention propose précisément des composés de ce type, dans lesquels plus particulièrement les points d'accrochage de deux ramifications sont différents et les ramifications sont présentes vers le milieu de la chaîne.

L'intérêt de ces composés à deux ramifications réside dans leur point de fusion et leur point d'écoulement très bas, beaucoup plus bas que ceux des dérivés ne comportant qu'une ramification.

Un intérêt particulier des groupements éthyles par rapport aux groupements méthyles réside dans la plus grande biodégradabilité des premiers.

Par ailleurs les domaines d'application des composés de l'invention, qui peuvent être considérés comme des acides isostéariques particuliers, sont ceux des acides isostéariques connus, notamment la fabrication de sels métalliques, d'émulsifiants, de plastifiants et de lubrifiants.

On considère dans l'invention les composés sous forme d'acides et d'esters d'alkyles inférieurs ou de glycéryle mais aussi les composés qui en dérivent, tels que des esters d'alkyles plus lourds, formés avec de nombreux alcools, des amides formés avec des amines, ainsi que des sels d'amines, des sels métalliques et des alkylolamides.

La nouvelle famille de composés chimiques de l'invention peut être définie par les formules suivantes :

$$(C_2H_5)_2C_{17}H_{29}COOR \qquad (I)$$

$$(C_2H_5)(C_2H_4)C_{17}H_{30}COOR \qquad (II)$$

$$(C_2H_5)(C_2H_3)C_{17}H_{31}COOR \qquad (III)$$

$$(C_2H_5)_2C_{17}H_{31}COOR \qquad (IV)$$

$$(C_2H_5)(C_2H_4)C_{17}H_{32}COOR \qquad (V)$$

$$(C_2H_5)_2C_{17}H_{33}COOR \qquad (VI)$$

La fixation des groupements $C_2H_5$, $C_2H_4$ et/ou $C_2H_3$ sur la chaîne est schématisée par les structures a, b et c ci-après.

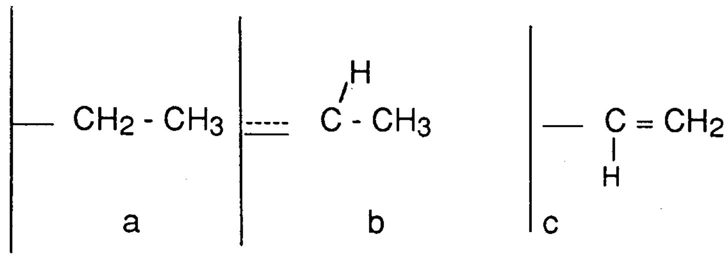

Les positions des différents groupes a, b, c sont en 9-11, 9-12, 10-12 ou 10-13.

R est un atome d'hydrogène, un radical alkyle tel que $CH_3$ ou $C_2H_5$,.ou un radical glycéryle.

Les composés répondants aux formules (IV), (V) et (VI) sont obtenus respectivement, par hydrogénation partielle ou totale des composés de formules (I), (II) et (III).

Les corps gras qui peuvent être engagés dans la réaction avec l'éthylène selon l'invention sont par exemple des huiles polyinsaturées comme les huiles de tournesol, de soja de carthame, des huiles de colza, de préférence des huiles riches en diènes ou triènes, mais aussi les dérivés de ces huiles comme les acides gras di-insaturés et les esters méthyliques des huiles. On peut aussi, de préférence, engager des composés di-insaturés conjugués. La conjugaison s'obtient soit avec les complexes au rhodium, soit par des alcoolates de potassium qui généralement, selon un procédé connu, donnent des isomères 9-11 ct et 10-12 tc.

Le catalyseur utilisé est à base de rhodium, mais seule une certaine famille de complexes permet d'obtenir cette addition de deux groupements éthylènes à des endroits différents de la chaîne.

Cette famille spécifique de catalyseurs au rhodium se caractérise par la formule suivante :

$$[Rh\ X_4]^{\ominus}\ [YR_4]^{\oplus}$$

dans laquelle chaque X représente un atome d'halogène Cl,Br ou F, un groupe $OH^-$, $OR^-$, $R^-$, $SO_3$ R ou $SO_4$ avec au moins un X = halogène, Y représente un atome d'azote ou de phosphore et R' représente un radical alkyle, cycloalkyle, aryle, alkylaryle, alcényle, ou acyle, ou un groupe polymèrique tel que le polystyrène.

Dans la formule (VII), les X peuvent être différents entre eux mais l'un au moins doit être un halogénure.

Les groupements R' peuvent aussi être différents entre eux.

Parmi les composés de sel quaternaire, on peut citer plus particulièrement: :

chlorure, l'octadécyl triméthyl ammonium chlorure, le didécyl diméthyl ammonium chlorure, le lauryl pyridinium chlorure, le diméthyl dibenzyl ammonium chlorure et le diméthyl stéaryl benzyl ammonium chlorure.

La plupart des sels quaternaires définis ci-dessus sont très répandus et bon marché, car ils constituent les matières premières pour des détergents, des adoucissants ou des bactéricides.

Le contre-ion est plus particulièrement un sulfate, un sulfonate, ou de préférence pour la réaction, un halogénure.

Un cas plus complexe est celui des sels quaternaires avec un groupement polymérique (qui sont par exemple vendus par Flucka) à base d'ammonium ou de phosphonium avec un groupement polystyrène.

Enfin, il existe un dérivé phosphorane $PØ_3$ = CH-CO-Ø non ionique, qui aprés réaction avec Rh Cl3, peut former un complexe ionique de ce type

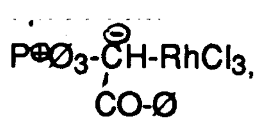

Ø représentant un radical phényle.

Les conditions de réaction sont assez souples. La pression d'éthylène peut varier par exemple de 0,1 à 30 MPa, de préférence de 0,3 à 4 MPa, la température par exemple de 20 à 160 °C, de préférence de 90 à 120 °C, les temps étant variables de 1h à 24h. Les concentrations de complexe de rhodium, lorsqu'on travaille avec un catalyseur homogène sont par exemple dans un rapport molaire avec le substrat de corps gras de l'ordre de 1/50 à 1/5000.

On peut réaliser la réaction d'addition de l'éthylène en discontinu et récupérer à la fin de la réaction le catalyseur avec une terre activée ou un polymère basique à base de résines.

Après élimination du catalyseur, on peut hydrogéner les composés diramifiés par exemple avec des catalyseurs au

nickel, au palladium ou au platine et même avec le même catalyseur.

Si le catalyseur est sur polymère, il est possible de le filtrer et de le réutiliser plusieurs fois de suite.

Le catalyseur est dans un premier temps formé par addition de 1 à 4 moles de sels quaternaires sur un composé du rhodium III, généralement sur un chlorure ou un bromure de rhodium. Cette réaction se fait généralement dans un solvant que l'on peut évaporer par la suite. La réaction peut être formulée comme suit :

$$RhX_3 + {}^{\oplus}Y\,R'_4\,X \rightarrow [Rh\,X_4^{\ominus}]\,\overset{\oplus}{Y}\,R'_4$$

Ces composés du rhodium ont été amplement décrits par exemple par Blum dans l'hydrogénation de composés divers (cf. Blum Tetrahedron letters <u>24</u>, 4139-4142 (1983)).

La réaction peut aussi se dérouler de façon biphasique en présence d'un solvant hydrophile.

Par rapport à une réaction avec du chlorure de rhodium ou des composés phosphiniques ou des phosphites, on a pu constater une accélération de la vitesse d'addition de l'éthylène.

La partie décomposée du complexe de rhodium donne des traces de composé isobuténylé.

Par rapport à une réaction avec les complexes non ioniques, on peut obtenir des vitesses de réaction 100 fois plus grandes.

Les exemples présentés ci-après illustrent l'invention.

**<u>EXEMPLE 1</u>**

Dans un ballon de 100 ml contenant 20 ml de chloroforme, on introduit, sous argon, 0,22 g (0,74 mmol) de $PBu_4Cl$ (tétrabutyl chlorure de phosphonium). Après dissolution de ce sel, on ajoute 0,2 g de $RhCl_3.3H_2O$ (trichlorure de rhodium tri-hydrate) et 1 ml de méthanol. A la température ambiante, on agite environ 30 min. On obtient une solution rougeâtre qui est transparente. Il s'est formé du $RhCl_4^{-}$ - $\overset{+}{P}Bu_4$. Cette solution est injectée dans un autoclave (0,5 l) chargé de 100 ml (299 mmol) d'ester méthylique de l'huile de tournesol contenant 61 % de composés conjugués cis/trans obtenus par action du terbutylate de potassium sur un ester méthylique de tournesol à 130 °C. On chauffe à 50 °C. On charge 5 bars d'éthylène et sous lente agitation on continue à chauffer à 80 °C. Une fois cette température atteinte, on augmente la pression d'éthylène à 30 bars et on agite à 1500 t/min.

L'évolution de la réaction est suivie par des prélévements d'échantillons qui sont analysés par chromatographie en phase gazeuse (tableau 1) et par mesure de l'absorption d'éthylène. Si on hydrogène on obtient un chromatogramme plus simple.

TABLEAU 1

| RESULTAT DE L'EXEMPLE 1 AVEC FAIBLE AVANCEMENT EN PRODUIT 2:1 (2 éthylène/ester conjugué) | | | | | | | | |
|---|---|---|---|---|---|---|---|---|
| Echantillon N° | T min | $C_{18:2}$ ct % pds | $C_{18:2}$ tt % pds | A % pds | B % pds | C % pds | D % pds | E % pds |
| 1 | 57 | 9 | 15 | 60,4 | 0 | 15 | 0 | 0 |
| 2 | 131 | 1 | 7 | 47,7 | 3,2 | 25,5 | 14 | 1,4 |
| 3 | 228 | 1 | 5 | 32 | 4,5 | 27,5 | 27 | 3 |
| 4 | 307 | 0 | 3,8 | 21,9 | 5,2 | 27,8 | 36 | 5,3 |

Les résultats indiqués se rapportent à l'ester conjugué de l'acide linoléique, au départ, de 100 %.

A : isomères vinyliques non conjugués du produit 1:1 (1 ramification),
B : isomères du produit 1:1 (1 ramification),
C : isomères conjugués du produit 1:1 (1 ramification),
D : isomères du produit 2:1 à double ramification,
E : isomères du produit 2:1 à une ramification isobutylique,
ct : cis-trans, tt : trans-trans.

On constate qu'avec une conversion de 96 % on obtient dans cet exemple 36 % de produit 2:1 à double ramification éthylènique (échantillon 4; produit D).

Le poids moléculaire mesuré en spectroscopie de masse, égale 354 après hydrogénation. L'analyse de ces dérivés a été obtenue par chromatographie en phase gazeuse couplée à un spectroscope. Les composés de PM = 354

sont d'après la spectroscopie de masse des composés diéthyl-stéarate comme on peut le voir dans les spectres des isomères par analyse des fragments. On donne aux figures 1 et 1A un exemple de spectre pour un des composés. On constate que les composés diéthyl- stéarates se comportent en fragmentation très différemment des composés monoramifiés, si l'on considère les deux structures schématisées par A et B ci-après

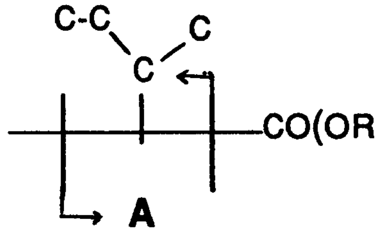

**A**

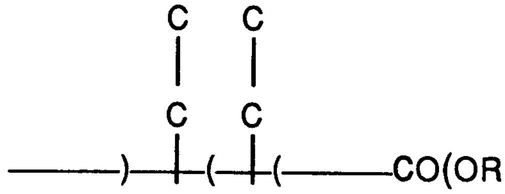

**B**

La fragmentation d'un composé mono-ramifié comme l'indique D.G. CHASSIN, Chem. Phys. Lipids 6 8-30 (1971) devrait se faire selon le schéma donné ci-dessus en A. Le spectre de masse est donc très différent de celui du produit B, où on peut déceler des fragments plus nombreux.

On donne ci-après des exemples plus détaillés pour un dérivé diéthyle et pour un dérivé isobutyle, à savoir le 9,12-diéthyl stéarate et le 10-isobutyl stéarate de méthyle. (voir tableaux 2 et 3).

TABLEAU 2

| Fragments obtenus par spectroscopie de masse du dérivé 9,12-diéthylstéarate de méthyle. | |
|---|---|
| M | = 354 |
| M - A | = 325 |
| B - A | = 293 |
| M - C | = 269 |
| B - C | = 237 |
| M - E | = 227 |
| M - K | = 213 |
| B-(A+C) | = 207 |
| B - E | = 195 |
| J | = 197 |
| M - G | = 199 |
| B - K | = 181 |
| B - G | = 167 |
| M - I | = 157 |

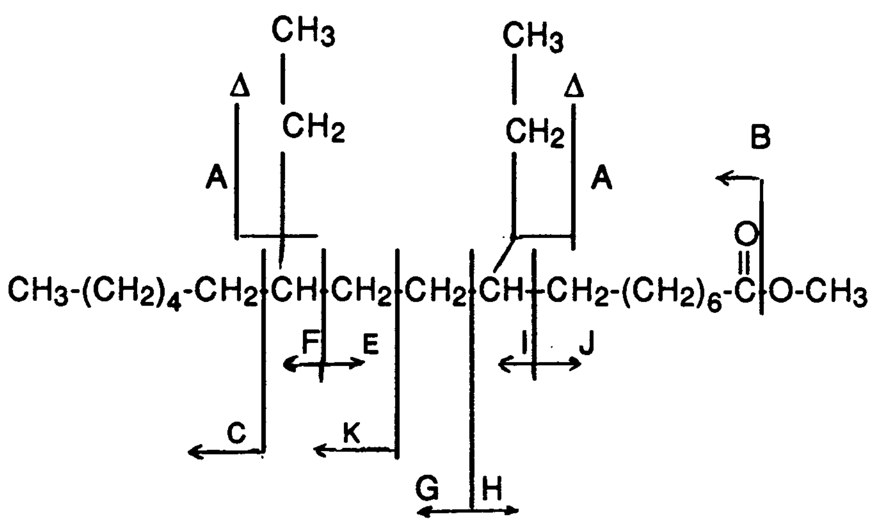

Le spectre correspondant est présenté sur les figures 1 et 1A.

TABLEAU 3

| Fragments obtenus par spectroscopie de masse d'un 10-isobutyl stéarate de méthyle. | |
|---|---|
| M | = 354 |
| M - A | = 297 |
| B - A | = 265 |
| M - C | = 241 |
| M - E | = 171 |
| B - C | = 209 |
| M - T | = 281 |

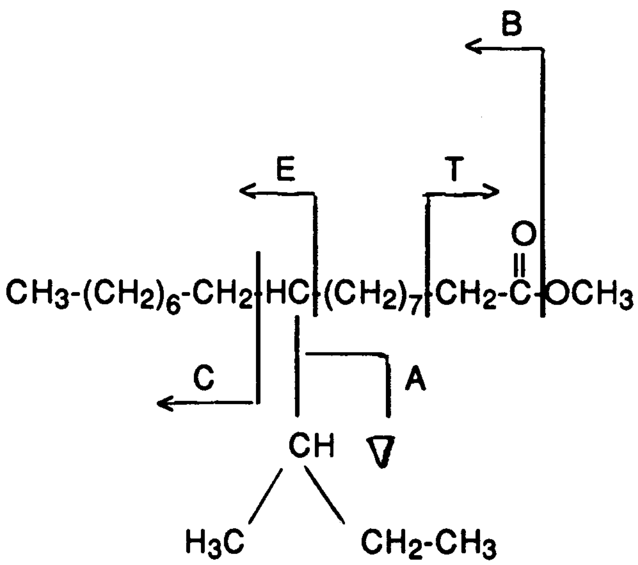

On a montré sur le tableau 3, que les fragments correspondent à pratiquement toutes les coupures possibles. Dans le spectre réel, on retrouve les mêmes fragments (voir le spectre correspondant présenté sur les figures 2 et 2A).

Pour tous les autres pics correspondants aux isomères de poids moléculaire 354 on constate que les fragmentations correspondent aux positions diéthyle en 10-13, 10-12, 9-12 et 9-11 (Figures 1 et 1A). Lorsqu'on a un isobutyle, on constate un départ d'un fragment important de 57 comme l'indique le tableau et le spectre de masse d'un dérivé isobutyle (Figures 2 et 2A). Il n'y a pas toutes les coupures rencontrées pour le dérivé diéthyl stéarate.

Le dérivé diéthyl stéarate de méthyle a des propriétés particulièrement intéressantes. Le point de fusion du mélange est de -55 °C donc particulièrement bas. Sous la forme acide, le point de fusion est inférieur à -18 °C.

Tous les composés diéthyles se caractérisent par une perte au départ d'un fragment éthyle important, ce qui n'est pas le cas avec l'isobutyle.

**EXEMPLE 2**

On répète l'exemple 1 sauf que la pression d'éthylène est ici de 2MPa.

TABLEAU 2

| N° | T h | $C_{18:2}$ ct % poids | $C_{18:2}$ tt % poids | A % poids | B % poids | C % poids | D % poids | E % poids |
|---|---|---|---|---|---|---|---|---|
| 1 | 2,25 | 3 | 6 | 56,8 | 2 | 21 | 11 | 0 |
| 2 | 4 | 0 | 2,8 | 34 | 4,5 | 28 | 29 | 1,7 |
| ct= cis-trans tt = trans-trans | | | | | | | | |

Les produits A, B, C, D, E correspondent aux produits désignés dans l'exemple 1.
On constate ici que le composé isobutyle fait moins de 2 %. (échantillon 2; Produit E).

**EXEMPLE 3**

Dans cet exemple, on synthétise le catalyseur à partir de 1,48 mmol de trichlorure de rhodium trihydraté et de 1,48 mmol de tétrabutyl chlorure de phosphonium.

300 mmol de $C_{18:2}$ conjugué cis/trans sont utilisés. Les conditions de réaction sont de 90 °C et 2MPa d'éthylène. On ne compte ici que l'ester conjugué.

TABLEAU 3

| N° | T h | $C_{18:2}$ ct % poids | $C_{18:2}$ tt % poids | A % poids | B % poids | C % poids | D % poids | E % poids |
|---|---|---|---|---|---|---|---|---|
| 1 | 3 | 0 | 0,5 | 33,7 | 6,5 | 24,7 | 32 | 2,6 |
| 2 | 5 | 0 | 0 | 29 | 6,5 | 25 | 35 | 3,5 |

**EXEMPLE 4**

On repète l'exemple 3 mais en utilisant 91 mmol de $C_{18:2}$ conjugué pour 1,48 mmol de $[RhCl_4]^{\ominus}$ $[PBu_4]^{\oplus}$ mais en analysant plus tôt les échantillons.

TABLEAU 4

| N° | T min | $C_{18:2}$ ct % poids | $C_{18:2}$ tt % poids | A % poids | B % poids | C % poids | D % poids | E % poids |
|---|---|---|---|---|---|---|---|---|
| 1 | 49 | 8,4 | 16 | 60 | 1 | 9 | 3,9 | 0 |
| 2 | 104 | 0 | 0 | 30 | 4,8 | 32 | 29,4 | 2 |
| 3 | 184 | 0 | 0 | 13 | 6 | 30 | 46 | 3 |
| 4 | 284 | 0 | 0 | 8 | 5 | 30 | 52 | 3 |

On constate qu'après 50 minutes on à déjà 60 % d'un dérivé mono-ramifié et aucune trace d'isobutényle. Le dérivé mono-ramifié se convertit en di-ramifié.

**EXEMPLE 5**

Dans cet exemple, on utilise 100g du produit d'une réaction d'addition d'éthylène suivant les conditions de l'exemple 1 dont la composition est donnée par l'échantillon N° 0 du tableau suivant.

Le produit initial contenait 184 mmol de composés conjugués (C18 : 2 et tt). La solution catalytique est constituée de 1,85 mmol de $RhCl_3$, $3H_2O$ et de 1,85 mmol $PBu_4Cl$.

Les conditions réactionnelles sont les mêmes que celles de l'exemple 1.

TABLEAU 5

| N° | T min | $C_{18:2}$ ct % poids | $C_{18:2}$ tt % poids | A % poids | B % poids | C % poids | D % poids | E % poids |
|---|---|---|---|---|---|---|---|---|
| 0 | 0 | 15 | 5,4 | 42,6 | 1,7 | 29 | 5,5 | 0 |
| 1 | 150 | 0 | 0 | 6 | 4,7 | 30 | 56 | 2 |
| 2 | 230 | 0 | 0 | 3 | 3,5 | 24,7 | 65 | 3 |
| 3 | 280 | 0 | 0 | 0 | 2,7 | 19 | 74 | 4 |
| On voit ici que la conversion en dérivé di-ramifié est de 74 %. | | | | | | | | |

**EXEMPLE 6**

On hydrogène au palladium sur charbon à 100 °C un produit obtenu comme décrit dans l'exemple 5 par le complexe $[RhCl_4]^{\ominus}[PBu_4]^{\oplus}$. Le produit de départ contient de l'oléate de méthyle (ester monoinsaturé en C18). Après hydrogénation, le stéarate formé et le palmitate également présent dans le substrat de départ donnent un mélange dont le point de trouble est de - 10°C.
L'élimination par cristallisation dans l'acétone de ces esters saturés permet alors d'obtenir un mélange dont le point de trouble est aussi bas que - 55°C.
L'hydrolyse des esters purifiés conduit à la forme acide qui consiste essentiellement en un mélange d'acides monoéthyl-et diéthyl-stéariques dont le point de trouble est encore assez bas (-18°C).

La composition d'un mélange d'esters méthyliques obtenu après estérification du mélange d'acides cristallisé est la suivante :

Produit 1:1 = 25% en poids
Produit 2:1 diéthyl = 75% en poids
Produit 2:1 isobutyl = traces

**EXEMPLE 7**

Dans cet exemple, on part de l'huile de tournesol avec un système identique à celui de l'exemple 1.

TABLEAU 7

| N° | T min | $C_{18:2}$ % poids | $C_{18:2}$ ct % poids | $C_{18:2}$ tt % poids | Produit 1:1 % poids | Produit 2:1 % poids |
|---|---|---|---|---|---|---|
| 1 | 148 | 73,3 | 2,6 | 3,2 | 16,3 | 4,6 |
| 2* | 293 | 47,4 | 0 | 0 | 15,6 | 37 |

* Injection d'une nouvelle solution catalytique (0,37 mmol).

Les résultats sont rapportés aux 69 % poids de $C_{18:2}$ contenu dans l'huile de tournesol.

Il est à remarquer que la sélectivité en produit d'addition de deux moles d'éthylène pour un ester est relativement élevée par rapport aux réactions avec l'ester. Par contre, la réaction est beaucoup plus lente quoique beaucoup plus rapide qu'avec du chlorure de rhodium.

**EXEMPLE 8**

Dans cet exemple on utilise 1,48 mmol de trichlorure de rhodium trihydraté et 1,48 mmol de diméthyl-benzyl-stéaryl chlorure d'ammonium dans 20 ml de chloroforme. On laisse réagir 50 ml d'huile de tournesol dans 60 ml d'heptane, en présence du catalyseur sous 2MPa d'éthylène à 90 °C. Après 7h de temps de réaction, on hydrogène le produit de la réaction et on obtient : 19 % poids de produit 1:1 et 51 % en poids de produit 2:1.

## Revendications

1. Composé organique di-ramifié diénique comportant une chaîne hydrocarbonée linéaire de 18 atomes de carbone, caractérisé en ce que ladite chaîne hydrocarbonée linéaire porte deux chaînes latérales chacune de 2 atomes de carbone en positions 9-11, 9-12, 10-12 ou 10-13, et en ce qu'il répond à l'une des formules générales :

$$(C_2H_5)_2C_{17}H_{29}COOR \quad (I)$$

$$(C_2H_5)(C_2H_4)C_{17}H_{30}COOR \quad (II)$$

$$\text{et } (C_2H_5)(C_2H_3)C_{17}H_{31}COOR \quad (III)$$

dans lesquelles R représente un atome d'hydrogène, un radical alkyle ou un radical glycéryle.

2. Composé organique di-ramifié, dérivant d'un composé selon la revendication 1 par hydrogénation partielle, caractérisé en ce qu'il répond à l'une des formules générales :

$$(C_2H_5)_2C_{17}H_{31}COOR \quad (IV)$$

$$(C_2H_5)(C_2H_4)C_{17}H_{32}COOR \quad (V)$$

dans lesquelles les chaînes latérales de 2 atomes de carbone sont en positions 9-11, 9-12, 10-12 ou 10-13 et R est défini comme dans la revendication 1.

3. Composé organique di-ramifié, dérivant d'un composé selon la revendication 1 ou 2, par hydrogénation totale, caractérisé en ce qu'il répond à la formule générale :

$$(C_2H_5)_2C_{17}H_{33}COOR \quad (VI)$$

dans laquelle les chaînes latérales de 2 atomes de carbone sont en positions 9-11, 9-12, 10-12 ou 10-13 et R est défini comme dans la revendication 1.

4. Procédé de fabrication d'un composé selon la revendication 1, caractérisé en ce que l'on fait réagir de l'éthylène avec au moins un acide carboxylique à chaîne linoléique, éventuellement conjugué, ou au moins un ester d'alkyle ou de glycéryle d'au moins un tel acide, en une proportion d'environ 2 moles d'éthylène par chaîne linoléique du dit acide ou ester, en présence d'un catalyseur au rhodium anionique représenté par la formule

$$[Rh\ X_4]^{\ominus}\ [YR'_4]^{\oplus}$$

dans laquelle chaque X représente un atome d'halogène, Cl, Br ou F, un groupe $OH^-$, $OR'^-$, $R'^-$, $SO_3$ R' ou $SO_4$ , avec au moins un X = halogène, Y représente un atome d'azote ou de phosphore et R' représente un radical alkyle, cycloalkyle, aryle, alkylaryle, alcényle, ou acyle, ou un groupe polymèrique tel que le polystyrène.

5. Procédé selon la revendication 4, caractérisé en ce que ladite réaction est réalisée à une température de 20 à 160 °C et à une pression de 0,1 à 30 MPa.

6. Procédé selon l'une des revendications 4 et 5, caractérisé en ce que ledit catalyseur au rhodium anionique est choisi parmi les composés de formules :

$$[Rh\ Cl_4^{\ominus}]\ \overset{\oplus}{N}\ Bu_4 \text{ et } [RhC\overset{\ominus}{l}_4]\ \overset{\oplus}{N}(Me_2)(St)B_zl$$

où Bu représente un radical butyle, Me un radical méthyle, St un radical octadecyl et Bzl un radical benzyl.

7. Procédé selon l'une des revendications 4 et 5, caractérisé en ce que dans ledit catalyseur au rhodium anionique, le rhodium est supporté sur un complexe anionique contenant un ligand polystyrène.

8. Procédé selon la revendication 4 et 5, caractérisé en ce que ledit catalyseur au rhodium anionique est choisi parmi des composés de formules :

$[Rh\,Cl_4^{\ominus}]\,P^{\oplus}Bu_4$

9. Procédé de fabrication d'un composé selon la revendication 2, caractérisé en ce que l'on soumet au moins un composé selon la revendication 1 à une hydrogénation partielle, en présence d'un catalyseur d'hydrogénation.

10. Procédé de fabrication d'un composé selon la revendication 3, caractérisé en ce que l'on soumet au moins un composé selon l'une des revendications 1 et 2 à une hydrogénation totale, en présence d'un catalyseur d'hydrogénation.

11. Procédé selon la revendication 10, caractérisé en ce que le produit obtenu substantiellement sous forme d'acide diéthyl-stéarique ou de diéthyl-stéarate d'alkyle, est soumis à une cristallisation à basse température dans un solvant, de manière à le séparer des composés stéariques, monoéthylstéariques et des composés palmitiques éventuellement présents.

12. Utilisation d'au moins un composé selon l'une des revendications 1 à 3, comme base de lubrifiant ou d'émulsifiant.

13. Utilisation d'au moins un composé selon l'une des revendications 1 à 3 comme base de lubrifiant ou d'émulsifiant après transformation des fonctions acides, esters d'alkyle ou de glycéryle en fonctions amides, sels d'amines, sels métalliques ou alkylolamides.

14. Composé organique dérivant d'au moins un composé selon l'une des revendications 1 à 3, par transformation des fonctions acides ou esters d'alkyle ou de glycéryle, en fonctions amides, sels d'amines, sels métalliques ou alkylolamides.

## Claims

1. Double-branched organic dienic compound comprising a linear hydrocarbon chain having 18 carbon atoms, characterized in that said linear hydrocarbon chain carries two lateral chains each with 2 carbon atoms in 9-11, 9-12, 10-12 or 10-13 positions, and in that it has one of the following general formulae :
general formulae:

$$(C_2H_5)_2C_{17}H_{29}COOR \qquad (I)$$

$$(C_2H_5)(C_2H_4)C_{17}H_{30}COOR \qquad (II)$$

$$\text{and } (C_2H_5)(C_2H_3)C_{17}H_{31}COOR \qquad (III)$$

where R represents a hydrogen atom, an alkyl radical or a glyceryl radical.

2. Double-branched organic compound derived by partial hydrogenation from a compound according to claim 1, characterized in that it has one of the following general formulae:

$$(C_2H_5)_2C_{17}H_{31}COOR \qquad (IV)$$

$$(C_2H_5)(C_2H_4)C_{17}H_{32}COOR \qquad (V)$$

where the lateral chain with 2 carbon atoms are in 9-11, 9-12,10-12 or 10-13 positions and R is as defined in claim 1.

3. Double-branched organic compound derived by total hydrogenation from a compound according to claim 1 or 2, characterized in that it has the following general formula:

$$C_2H_5)_2C_{17}H_{33}COOR \qquad (VI)$$

where the lateral chain with 2 carbon atoms are in 9-11, 9-12,10-12 or 10-13 positions and R is as defined in claim 1.

4. Process for the production of a compound according to claim 1, characterized in that ethylene is reacted with at least one carboxylic acid having a linoleic chain which may optionally be conjugated, or at least one alkyl or glyceryl

ester of at least one such acid, in proportions of about 2 moles of ethylene per linoleic chain of said acid or ester, in the presence of an anionic rhodium catalyst represented by the formula

$$[RhX_4]^-[YR_4]^+$$

where each X represents a halogen atom, Cl, Br or F, or a $OH^-$, $OR^-$,.$R^{--}$, $SO_3$-R' or $SO_4^{--}$ group, with at least one X being a halogen, Y represents a nitrogen.

5. Process according to claim 4, characterized in that said reaction is carried out at a temperature of 20°C to 160°C and pressure of 0.1 to 30 MPa. atom or a phosphorus atom and R' represents an alkyl, cycloalkyl, aryl, alkylaryl, alkenyl or aryl group or a polymer group such as polystyrene.

6. Process according to any one of claims 4 and 5, characterised in that said anionic rhodium catalyst is selected from compounds having formulae:

$$[RhCl_4]^- \, {}^+NBu_4 \text{ and } [RhCl_4]^- \, {}^+N(Me_2)(St)Bzl$$

where Bu represents a butyl radical, Me a methyl radical, St an octadecyl radical and Bzl a benzyl radical.

7. Process according to any one of claims 4 and 5, characterized in that, in said anionic rhodium catalyst, the rhodium is supported on an anionic complex containing a polystyrene ligand.

8. Process according to any one of claims 4 and 5 , characterised in that said anionic rhodium catalyst is selected from compounds with formulae:

$$[RhCl_4]^- \, {}^+PBu_4$$

9. Process for the production of a compound according to claim 2, characterized in that at least one compound according to claim 1 is partially hydrogenated, in the presence of a hydrogenation catalyst.

10. Process for the production of a compound according to claim 3, characterised in that at least one compound according to claims 1 and 2 is completely hydrogenated, in the presence of a hydrogenation catalyst.

11. Process according to claim 10, characterised in that the product obtained substantially in the form of the diethyl stearic acid or alkyl diethyl stearate is crystallized at low temperature from a solvent to separate the stearic, monoethylstearic and palmitic compounds which may be present.

12. Use of at least one compound according to any one of claims 1 to 3 as a lubricant or emulsifying agent base.

13. Use of at least one compound according to any one claims 1 to 3 as a lubricant or emulsifying agent base following transformation of the acid, alkyl or glyceryl ester functions into amides, amine salts, metallic salts or alkylolamides.

14. Organic compound derived from at least one compound according to claims 1 to 3, by transformation of the acid functions or alkyl or glyceryl esters into amides, amine salts, metallic salts or alkylolamides.

**Patentansprüche**

1. Doppelt verzweigte organische Dienverbindung, umfassend eine lineare Kohlenwasserstoffkette von 18 Kohlenstoffatomen, dadurch gekennzeichnet, daß die lineare Kohlenwasserstoffkette zwei Seitenketten mit jeweils zwei Kohlenstoffatomen in den Positionen 9-11, 9-12, 10-12 oder 10-13 trägt und daß sie einer der allgemeinen Formeln

$$(C_2H_5)_2C_{17}H_{29}COOR \quad (I)$$

$$(C_2H_5)(C_2H_4)C_{17}H_{30}COOR \quad (II)$$

$$\text{und } (C_2H_5)(C_2H_3)C_{17}H_{31}COOR \quad (III)$$

entspricht, worin R ein Wasserstoffatom, einen Alkylrest oder einen Glycerinrest darstellt.

2. Doppelt verzweigte organische Verbindung, die aus einer Verbindung nach Anspruch 1 durch partielle Hydrierung hergeleitet ist, dadurch gekennzeichnet, daß sie einer der allgemeinen Formeln

$$(C_2H_5)_2C_{17}H_{31}COOR \quad (IV)$$

$$(C_2H_5)(C_2H_4)C_{17}H_{32}COOR \quad (V)$$

entspricht, worin die Seitenketten von zwei Kohlenstoffatomen sich in den Positionen 9-11, 9-12, 10-12 oder 10-13 befinden und R wie in Anspruch 1 definiert ist.

3. Doppelt verzweigte organische Verbindung, die aus einer Verbindung nach Anspruch 1 oder 2 durch vollständige Hydrierung hergeleitet ist, dadurch gekennzeichnet, daß sie der allgemeinen Formel

$$(C_2H_5)_2C_{17}H_{33}COOR \quad (VI)$$

entspricht, worin die seitenketten von zwei Kohlenstoffatomen sich in den Positionen 9-11, 9-12, 10-12 oder 10-13 befinden und R wie in Anspruch 1 definiert ist.

4. Verfahren zur Herstellung einer Verbindung nach Anspruch 1, dadurch gekennzeichnet, daß man Ethylen mit wenigstens einer Carbonsäure mit Linolkette, welche gegebenenfalls konjugiert ist, oder wenigstens einem Alkyl- oder Glycerylester wenigstens einer solchen Säure in einem Verhältnis von etwa 2 Mol Ethylen pro Linolkette der Säure oder des Esters in Gegenwart eines Katalysators auf Basis von anionischem Rhodium umsetzt, der durch die Formel

$$[RhX_4]^-[YR'_4]^+$$

dargestellt ist, worin X jeweils ein Halogenatom, Cl, Br oder F, eine $OH^-$-, $OR'^-$-, $R'^-$-, $SO^3R'^-$- oder $SO_4^{2-}$-Gruppe darstellt, wobei wenigstens ein X ein Halogenatom ist, Y ein Stickstoff- oder Phosphoratom darstellt und R' ein Alkyl-, Cycloalkyl-, Aryl-, Alkylaryl-, Alkenyl- oder Acylrest oder eine polymere Gruppe, wie Polystyrol, ist.

5. Verfahren nach Anspruch 4, dadurch gekennzeichnet, daß die Umsetzung bei einer Temperatur von 20 bis 160°C und bei einem Druck von 0,1 bis 30 Mpa durchgeführt wird.

6. Verfahren nach einem der Ansprüche 4 und 5, dadurch gekennzeichnet, daß der Katalysator aus anionischem Rhodium aus den Verbindungen der Formeln

$$[RhCl_4]^-[NBu_4]^+ \text{ und } [RhCl_4]^-[N(Me_2)(St)Bzl]^+$$

ausgewählt ist, wobei Bu einen Butylrest, Me einen Methylrest, St ein Octadecylrest und Bzl ein Benzylrest ist.

7. Verfahren nach einem der Ansprüche 4 und 5, dadurch gekennzeichnet, daß bei dem Katalysator aus anionischem Rhodium das Rhodium auf einem anionischen Komplex getragen wird, der einen Polystyrolliganden enthält.

8. Verfahren nach Anspruch 4 und 5, dadurch gekennzeichnet, daß der Katalysator aus anionischem Rhodium aus den Verbindungen der Formeln

$$[RhCl_4]^-[PBu_4]^+$$

ausgewählt ist.

9. Verfahren zur Herstellung einer Verbindung nach Anspruch 2, dadurch gekennzeichnet, daß man wenigstens eine Verbindung nach Anspruch 1 einer teilweisen Hydrierung in Gegenwart eines Hydrierungskatalysators unterwirft.

10. Verfahren zur Herstellung einer Verbindung nach Anspruch 3, dadurch gekennzeichnet, daß man wenigstens eine Verbindung nach den Ansprüchen 1 und 2 einer vollständigen Hydrierung in Gegenwart eines Hydrierungskatalysators unterwirft.

11. Verfahren nach Anspruch 10, dadurch gekennzeichnet, daß das im wesentlichen in Form von Diethylstearylsäure oder Alkyldiethylstearat erhaltene Produkt einer Kristallisierung bei niedriger Temperatur in einem Lösungsmittel

unterworfen wird, um es von den gegebenenfalls vorhandenen Stearyl-, Monoethylstearylverbindungen und den Palmitinverbindungen abzutrennen.

**12.** Verwendung wenigstens einer Verbindung nach einem der Ansprüche 1 bis 3 als Schmier- oder Emulgiergrundstoff.

**13.** Verwendung wenigstens einer Verbindung nach einem der Ansprüche 1 bis 3 als Schmier- oder Emulgiergrundstoff nach Umwandlung der Säure-, Alkylester- oder Glycerylfunktionen in Funktionen von Amiden, Aminsalzen, Metallsalzen oder Alkylamiden.

**14.** Organische Verbindung, die aus wenigstens einer Verbindung nach einem der Ansprüche 1 bis 3 durch Umwandlung der Säure- oder Alkylester- oder Glycerylesterfunktionen zu Funktionen von Amiden, Aminsalzen, Metallsalzen ode Alkylamiden hergeleitet ist.

FIG.1

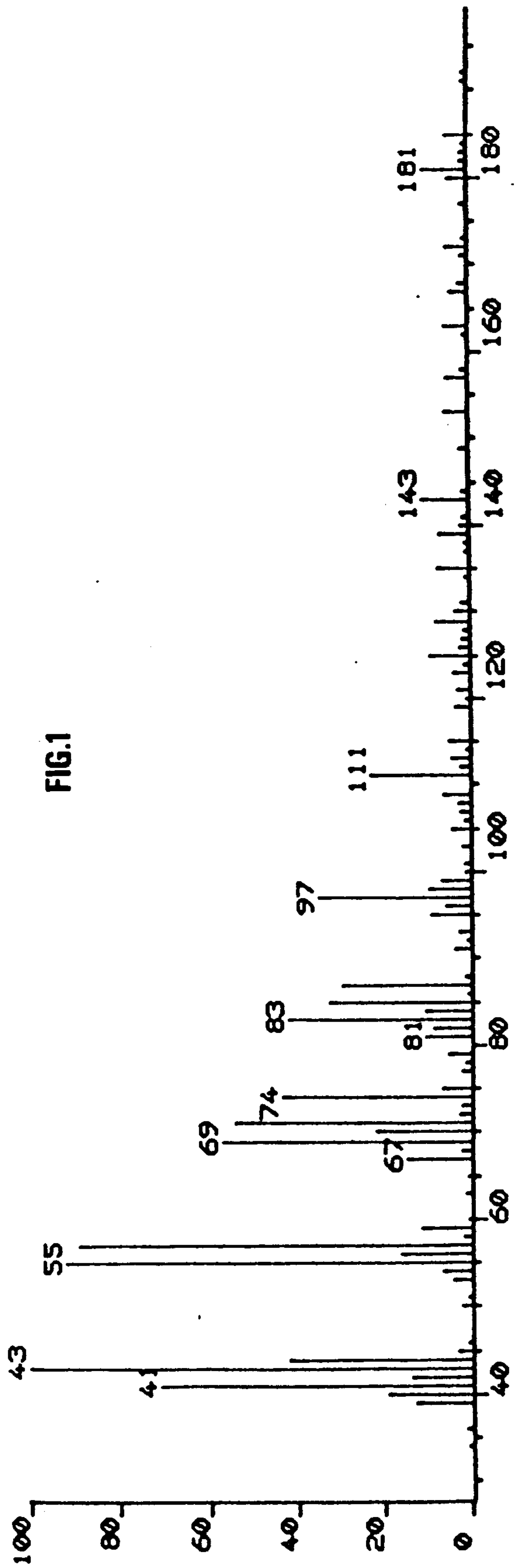

100
80
60
40
20
0
40
60
80
100
120
140
160
180
41
43
55
67
69
74
81
83
97
111
143
181


FIG.1A

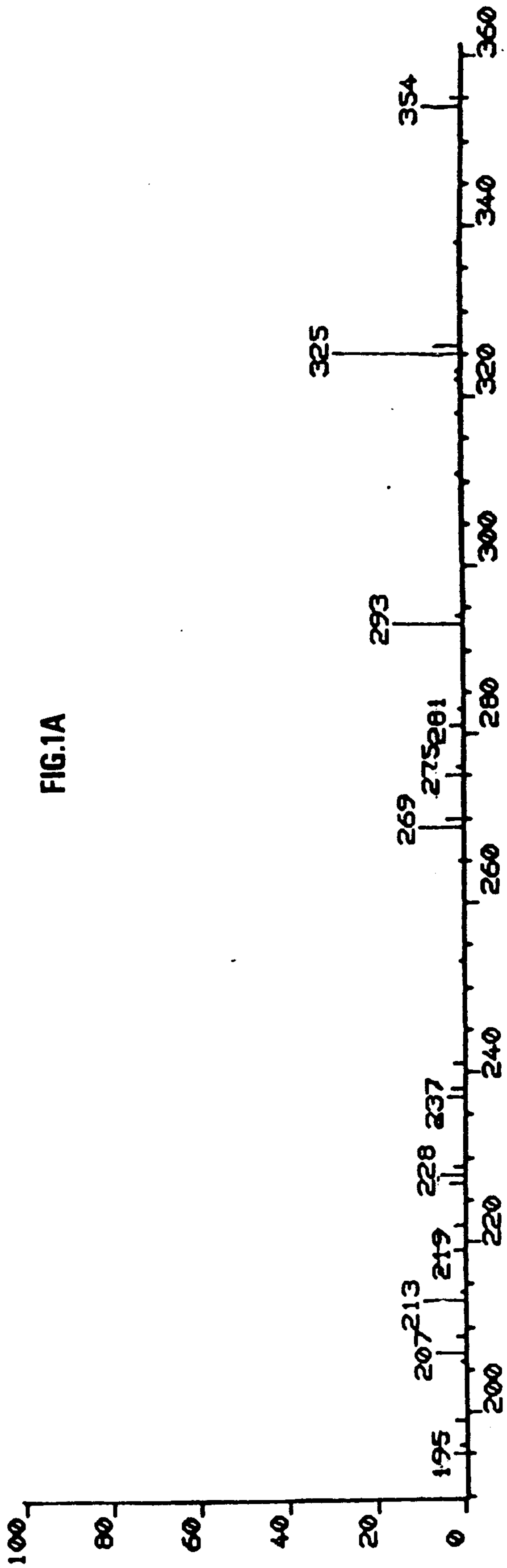

100
80
60
40
20
0
200
220
240
260
280
300
320
340
360
195
207
213
219
228
237
269
275
281
293
325
354

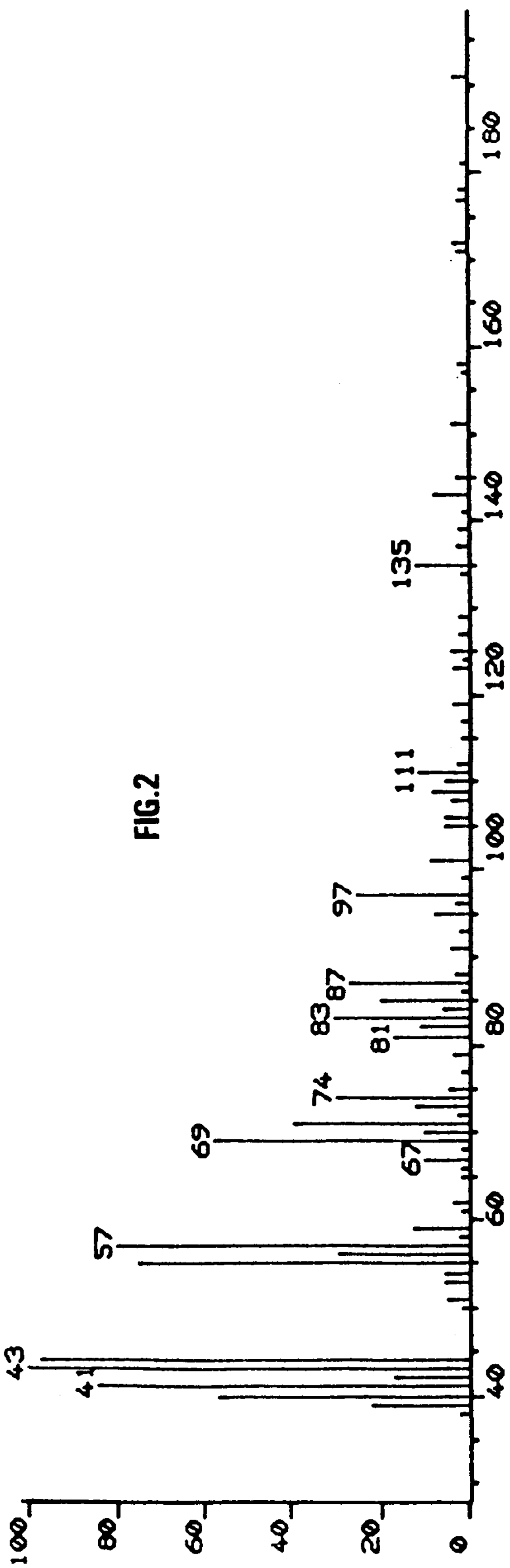

100
80
60
40
20
0
40
60
80
100
120
140
160
180
41
43
57
67
69
74
81
83
87
97
111
135


FIG.2

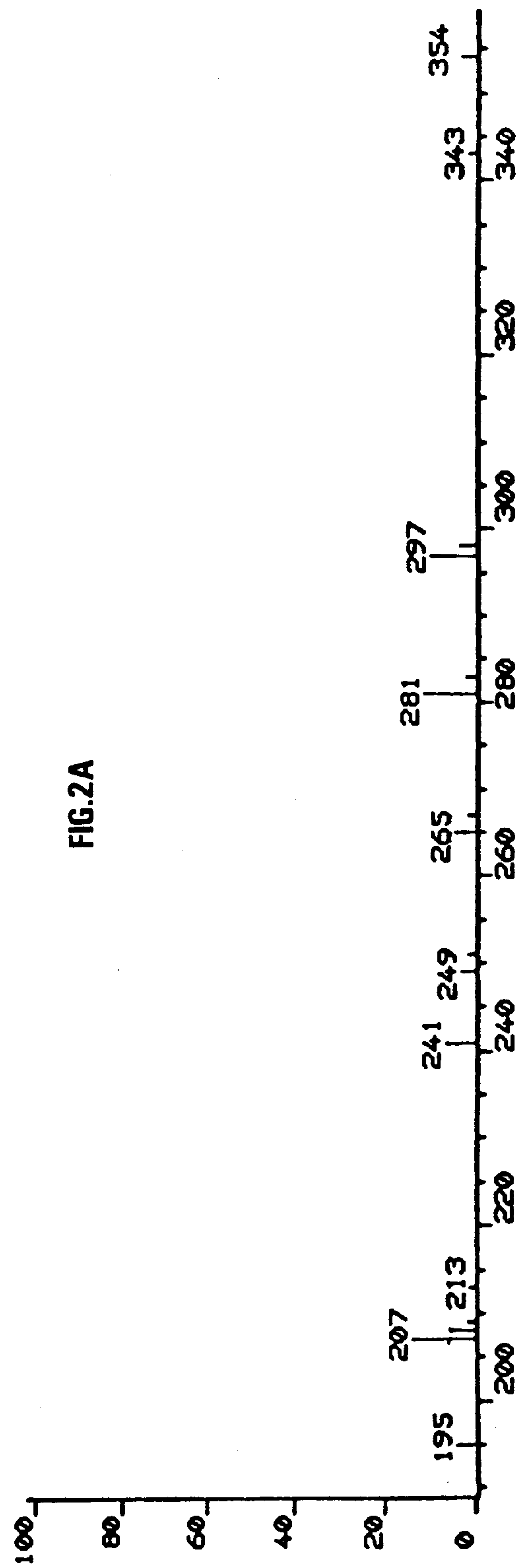

100
80
60
40
20
0
200
220
240
260
280
300
320
340
195
207
213
241
249
265
281
297
343
354


FIG.2A